Europäisches Patentamt

European Patent Office

Office européen des brevets

(11) Publication number: 0 219 214 B1

## EUROPEAN PATENT SPECIFICATION

(45) Date of publication of patent specification: **08.04.92** (51) Int. Cl.⁵: **C12N 15/85**

(21) Application number: **86306714.6**

(22) Date of filing: **29.08.86**

(54) Chimeric plasmid vector.

(30) Priority: **30.08.85 JP 191427/85**

(43) Date of publication of application:
**22.04.87 Bulletin 87/17**

(45) Publication of the grant of the patent:
**08.04.92 Bulletin 92/15**

(84) Designated Contracting States:
**AT BE CH DE FR GB IT LI LU NL SE**

(56) References cited:
**EP-A- 0 115 936**
**EP-A- 0 178 220**

**Cell, vol. 27, n 2 December 1981 (Part 1)
(Cambridge, USA). A.L. Huand et al
"Glucocorticoid regulation of the Ha-MuSV p
21 gene conferred by sequences from
mouse mammary tumor virus". Pages
245-255**

**Molecular and cellular biology, vol. 3, n 11,
November 1983 (Washington DC). M.C.
Ostrowski et al "Glucocorticoid regulation of
transcription at an amplified, episomal pro-
moter". Pages 2045-2057**

**Nature, vol. 294, n 5838, November 19, 1981**

**(New York, London). F. Lee et al
"Glucocorticoids regulate expression of
dihydrofolate reductase cDNA in mouse
mammary tumour virus chimaeric plas-
mids". Pages 228-232**

(73) Proprietor: **KABUSHIKI KAISHA YAKULT HON-
SHA
1-19, Higashishinbashi 1-chome
Minato-ku Tokyo 105(JP)**

(72) Inventor: **Shirasawa, Yukio
201, 2-1-7, Ojima Chofu
Tokyo(JP)**
Inventor: **Tsuchida, Nobuo
2-29-47, Kashiwa-cho Shiki
Saitama(JP)**

(74) Representative: **Brewer, Leonard Stuart et al
SANDERSON & CO. European Patent Attor-
neys 34, East Stockwell Street
Colchester Essex CO1 1ST(GB)**

## Description

### FIELD OF THE INVENTION

The present invention relates to a chimeric plasmid vector including a DNA sequence of an animal virus and, more particularly, to a chimeric plasmid vector which is useful for introducing a DNA fragment into animal cells for producing a desired protein product.

### BACKGROUND OF THE INVENTION

It is well known in the art that a biogenically active form of protein of a higher animal can be advantageously produced using gene manipulation techniques introducing a corresponding gene into the cell of the higher animal. This is because of the fact that most proteins of higher animals have modified structures having, for example, sugar-chain moieties, disulfide bonds and amidation of C-terminals.

For the expression of desired foreign genes in animal cells, it has been an ordinary practice to use animal viruses as vectors. When a vector having a transcription promotor of an animal virus linked with a foreign gene is used for introduction into an animal cell, the genetic information carried on the foreign gene is transcribed into the form of an RNA by the action of the viral promotor on the vector. The transcribed genetic information is translated from the RNA into the form of a protein molecule coded for by the genetic information originating in the foreign gene, as also well known in the art. Examples of animal viruses thus used as vectors include papovaviruses, papillomaviruses and retroviruses. Among these animal viruses, papovaviruse SV40 and a SV40-derived plasmid pSV2 and bovine papillomaviruses in particular have frequently been the subjects of research and investigations and have found a variety of practical applications as vectors. On the other hand, vectors using retroviruses have various unique advantages over vectors using other viruses and are expected to provide useful expedients for the future gene engineering. Such unique advantages of the vectors using retroviruses include:

(1) the wide host ranges which the vectors have for animal cells;

(2) the abilities of the vectors which can be incorporated into and stably maintained within host chromosomal DNAs;

(3) that the transfection with the vectors is not lethal to host animal cells; and

(4) the amenability of the vectors to insertion of relatively large foreign DNA fragments.

It is further well known in the art that the gene expression of a mouse mammary tumor virus (hereinafter referred to as MMTV), which is one of the retroviruses, is regulated, viz., strengthened by addition of glucocorticoid hormone. Taking advantage of this hormone dependency in the gene expression of the MMTV, attempts have thus far been made to synthesize vectors each carrying both a viral promotor region and a hormone dependent region thereof. Such a hormone dependent region, or hormone receptor complex binding region, of the MMTV is included in a "long terminal repeat" (LTR) region which also includes an MMTV promotor region. It is reported for example that a foreign ras gene can be effectively expressed to yield $p^{21}$ when linked with an LTR region of the MMTV (Cell, 27, 245-255, 1981). Other reports refer to an LTR-linked bovine papillomavirus vector (Mol. Cell. Biol., 3, 2045-2057, 1983) and an LTR-linked vector of SV40-derived pSV2 (Nature, 294, 228-232, 1981).

Each of these known LTR-linked vectors has only one LTR region therein and is accordingly devoid of an effectively acting transcription termination site. This means that the genetic information replicated from a foreign gene could not be transcribed into RNA accurately and in a stable condition. In respect of an LTR-linked vector, a problem is further encountered in that the transcription proceeds also for the DNA sequence intervening between the sole LTR region of the vector and the foreign gene region inserted into the vector downstream of the LTR region. For this reason, the transcription proceeds for the DNA sequence spanning from the promotor within the LTR region to the foreign gene region. Such an extended coverage of the transcription would result in instability of the transcription agent.

With a view to providing a solution to these problems and to exploiting the unique advantages of vectors derived from retroviruses, efforts have been made by the present inventors to develop new and useful chimeric plasmid vectors capable of producing proteins in animal cells accurately and in stable condition through the expression of the genetic information originally carried by foreign genes. The present invention is credited to such efforts and is based on the discovery that proteins can be produced stably in an animal cell by a chimeric plasmid vector which has at least two, first and second specific LTR regions. The first LTR region includes a hormone dependent region (or hormone receptor complex binding region) and an MMTV promotor region and the second LTR region is located downstream of the first LTR region with a polylinker carrying the insertion sites located between the two LTR regions. Between the polylinker and the first LTR region are provided a 5'-splicing site and a 3'-splicing site with the former located closer to the first LTR region and the latter located closer to the polylinker.

Thus, it is a prime object of the present invention to provide a novel chimeric plasmid vector which is specifically useful for producing a desired protein preferably having biogenic activities through accurate and stabilized expression of a foreign gene within animal cells.

## SUMMARY OF THE INVENTION

In accordance with the present invention, there is provided a chimeric plasmid vector which comprises first to fifth DNA fragments. The first DNA fragment has a first LTR region derived from an MMTV and a 5'-splicing site located downstream of the first LTR region, wherein the first LTR region comprises a hormone dependent region (or a hormone receptor complex binding region) and an MMTV promotor region located downstream of the hormone dependent region. The third DNA fragment has a second LTR region derived from the MMTV and having a transcription termination site. The first and third DNA fragments are linked with the fifth DNA fragment which comprises an ampicillin resistance gene region derived from plasmid pBR322 and a replication origin derived from the plasmid pBR322. A polylinker derived from plasmid pi-AN7 is inserted downstream of the first DNA fragment, whereupon the second DNA fragment which has a 3'-splicing site derived from plasmid pCVSVE is inserted immediately upstream of the polylinker. The fourth DNA fragment, which comprises an SV40 promotor region derived from plasmid pSV2gpt and an Eco-gpt gene region derived from the plasmid pSV2gpt, is then inserted as a gene marker between the fourth DNA fragment and the third DNA fragment.

## BRIEF DESCRIPTION OF THE DRAWINGS

The features and advantages of a chimeric plasmid vector according to the present invention will be more clearly appreciated from the following description taken in conjunction with the accompanying drawings in which:

Fig. 1 is a view showing first to fifth intermediate plasmids (pMM1 - pMM5) produced by some earlier steps of a process of synthesizing a chimeric plasmid vector (pM102) according to the present invention;

Fig. 2 is a view similar to Fig. 1 but shows fifth to ninth intermediate plasmids (pMM5 - pMM8) produced by steps subsequent to the steps shown in Fig. 1 as well as the chimeric plasmid vector (pMM102) resulting from the process shown in Figs. 1 and 2;

Fig. 3 is a schematic representation of a restriction enzyme cleavage map of the chimeric plasmid vector (pMM102) according to the present invention;

Fig. 3A is a schematic representation of the general structure composed of a total of five DNA fragments forming the chimeric plasmid vector (pMM102) shown in Fig. 3;

Fig. 4 is a view showing the nucleotide sequence of a polylinker which may be used in the chimeric plasmid vector according to the present invention; and

Fig. 5 is a view showing the palindromic nucleotide sequence of the double strand XhoI linker DNA used in the chimeric plasmid vector according to the present invention.

## DESCRIPTION OF THE PREFERRED EMBODIMENTS

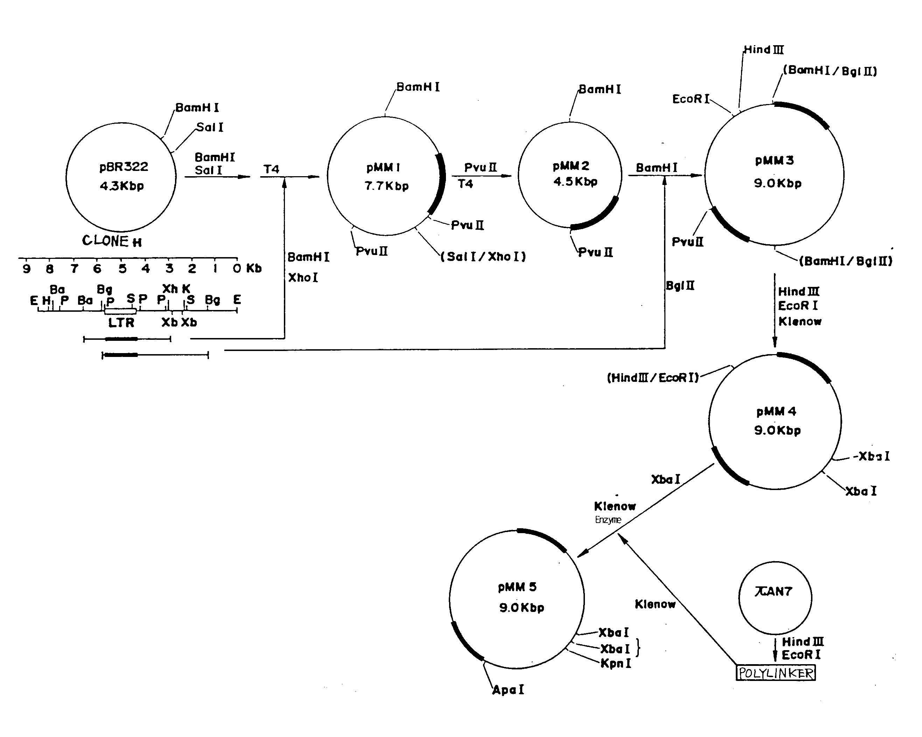

In a process of synthesizing a novel chimeric plasmid vector according to the present invention, an LTR-containing BamHI-XhoI digested fragment of clone H (Cell, 23, 335-345, 1981) is cloned at the BamHI-SalI cleavage site of E. coli plasmid pBR322 to create plasmid pMM1 as a first intermediate plasmid, as shown in Fig. 1. The clone H herein used has resulted from the cloning of MMTV at the EcoRI cleavage site of a lambda-phage vector lambda-gtWES .lambda-B (Cell, 23, 335-345, 1981). In Fig. 1, the alphabetic denotations used for the clone H refer to enzymes as follows:

Ba: BamHI; Bg: BglII; E: EcoRI; H: HindIII; K: KpnI; P: PstI; S: SstI; Xb: XbaI; Xh: XhoI.

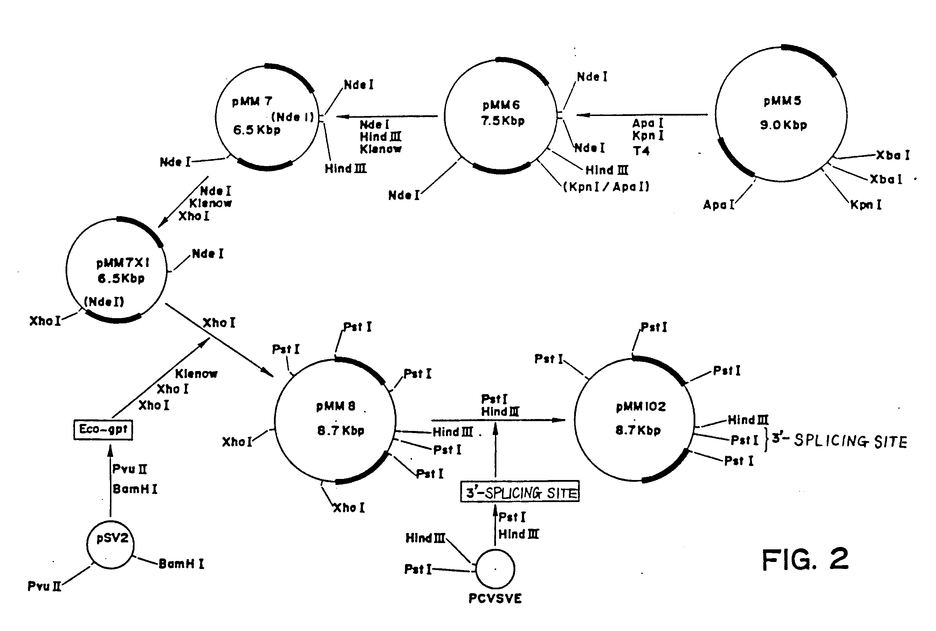

In Fig. 1 (and also in Fig. 2), furthermore, the restriction enzyme sites shown enclosed within parentheses are those cleavage sites which have been made extinct.

The first intermediate plasmid pMM1 thus created is cleaved with a restriction enzyme PvuII at the two PvuII cleavage sites of the plasmid, whereupon the larger one of the resultant two DNA fragments, viz., the fragment including the BamHI cleavage site is isolated from the other and is ligated with T4 DNA ligase to produce plasmid pMM2 as a second intermediate plasmid, as also shown in Fig. 1.

Subsequently, a BglII-digested DNA fragment containing the LTR region of the clone H is inserted at the BamHI cleavage site of the second intermediate plasmid pMM2 to yield plasmid pMM3 as a third intermediate plasmid. The third intermediate plasmid pMM3 is cleaved with restriction enzymes EcoRI and HindIII and the resultant DNA fragment is treated with Klenow enzyme to make its ends blunt and further with T4 DNA ligase so as to be circularized for thereby forming plasmid pMM4 as a fourth intermediate plasmid as shown in Fig. 1. It may be noted that the fourth intermediate plasmid pMM4 thus obtained has no EcoRI and HindIII cleavage sites for the ends of the cleaved fragment.

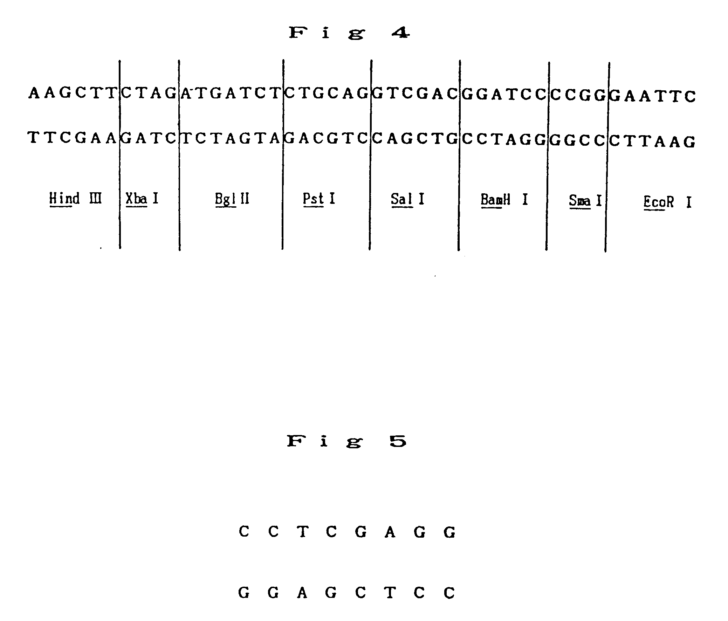

Such a fourth intermediate plasmid pMM4 is cleaved with a restriction enzyme XbaI and the resultant fragment is also treated with Klenow enzyme to make its ends blunt. The fragment is then circularized with its blunt ends linked together with a polylinker derived from a HindIII-EcoRI digested pi-AN7 fragment treated with Klenow enzyme, thereby producing plasmid pMM5 as a fifth intermediate plasmid as shown in Fig. 1 (Gene, 30, 257-260, 1984 for pi AN7). This polylinker consists of a DNA fragment having cleavage sites for restriction enzymes HindIII (AAGCTT), XbaI (CTAG), BglII (AGATCT), PstI (CTGCAG), SalI (GTCGAC), BamHI (GGATCC), SmaI (CCGG), EcoRI (GAATTC), as shown in Fig. 4.

The fifth intermediate plasmid pMM5 is digested with restriction enzymes ApaI and KpnI and the resultant cleaved fragment is treated with T4 DNA polymerase to make its ends blunt and then with T4 DNA ligase so as to be circularized in shrunk form into plasmid pMM6 as a sixth intermediate plasmid as shown in Fig. 2. The sixth intermediate plasmid pMM6 thus produced is partially digested with a restriction enzyme NdeI for a short period of time. Of a number of DNA fragments produced by the cleavage of a plenty of sixth intermediate plasmid pMM6, the fragments each resulting from a plasmid cleaved at one of its three NdeI cleavage sites are isolated by electrophoresis against an agarose gel plate from the DNA fragments each resulting from a plasmid cleaved at two or three of its NdeI cleavage sites. The DNA fragments isolated in this manner are digested with a restriction enzyme HindIII and the resultant DNA fragments are treated with Klenow enzyme and further with T4 DNA ligase for being circularized in shrunk form. It may be noted that a number of DNA fragments thus isolated are broken down to two categories, a DNA fragment of one category having two NdeI cleavage sites with one NdeI cleavage site made extinct and a DNA fragment of the other category having three NdeI cleavage sites. Of these two kinds of plasmids, those each having two NdeI cleavage sites, one of which has been derived from the plasmid pBR322, are herein used each as a seventh intermediate plasmid pMM7 as shown in Fig. 2.

The seventh intermediate plasmid pMM7 thus having two NdeI cleavage sites is partially digested with a restriction enzyme NdeI and the resultant DNA fragment is treated with Klenow enzyme and further with T4 DNA ligase so as to be circularized in shrunk form with an XhoI linker inserted between the blunt ends of the fragment. Of the number of plasmids thus obtained, those each of which has the XhoI linker located at the NdeI cleavage site derived from the plasmid pBR322 is selectively extracted as an eighth intermediate plasmid pMM7X1 as shown in Fig. 2. Fig. 5 shows the palindromic nucleotide sequence of the double strand XhoI linker DNA herein used.

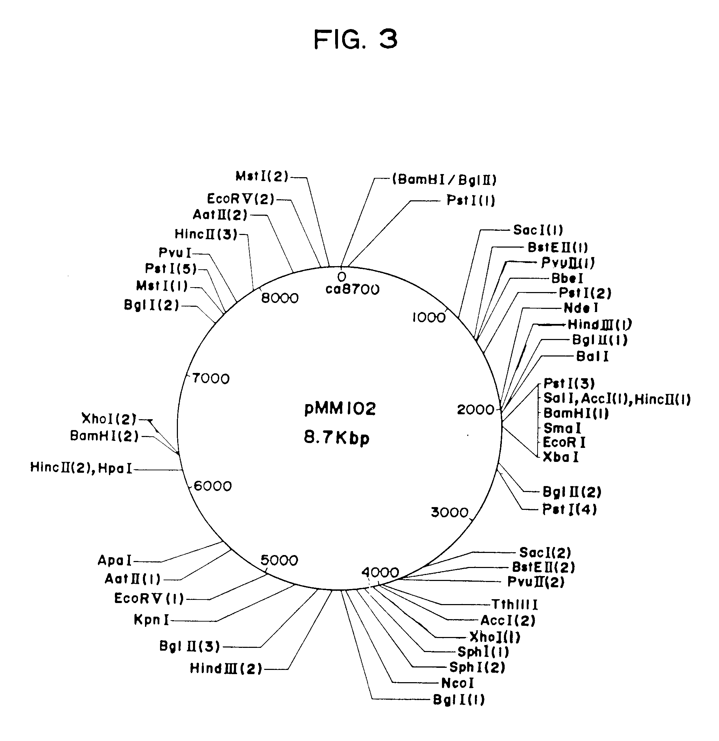

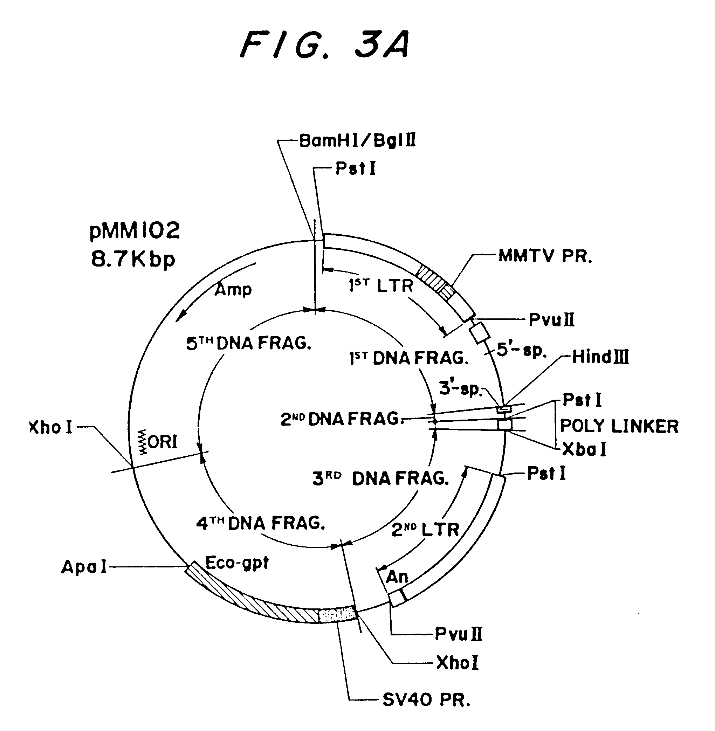

A PvuII-BamHI digested fragment of SV40-derived plasmid pSV2gpt (Science, 209, 1422-1427, 1980) containing an SV40 promotor region, an Eco-gpt gene (which inherently has myconophenolic acid resistance expressed) and an SV40 splicing site therein is treated with Klenow enzyme. An XhoI linker is linked to the resultant fragment, which is then inserted into the XhoI cleavage site of the eighth intermediate plasmid pMM7X1 to produce plasmid pMM8 as a ninth intermediate plasmid as shown in Fig. 2. The polylinker sites ranging between the HindIII and PstI cleavage sites of this ninth intermediate plasmid pMM8 is substituted by a HindIII-PstI digested DNA fragment containing a 3'-splicing site of plasmid pCVSVE (Mol. Cell. Biol., 2, 1304-1319, 1982), the 3'-splicing site of which has been derived from an immunoglobulin variable region. A chimeric plasmid vector according to the present invention is now finally synthesized, which is herein named pMM102. Fig. 3 represents a restriction enzyme cleavage map of the chimeric plasmid vector pMM102 thus provided in accordance with the present invention. Furthermore, Fig. 3A schematically represents the general structure composed of the first to fifth DNA fragments forming the chimeric plasmid vector (pMM102) according to the present invention.

As noted previously, the chimeric plasmid vector pMM102 according to the present invention can be replicated in E. coli. Such a novel chimeric plasmid vector is capable of transforming E.coli K-12 khi-1776 in a manner like that described in Example 1, and is carried on, for example, E.coli K-12 khi-1776 (pMM102) which has been deposited at Fermentation Research Institute, Agency of Industrial Science and Technology, Japan under the deposition number FERM P-8429.

The chimeric plasmid vector pMM102 according to the present invention is characterized inter alia in that the plasmid vector has two, first and second LTR regions as will be readily seen from Fig. 3A. The first LTR region, contained in the first DNA fragment, is derived from an MMTV and includes a hormone dependent region (or a hormone receptor complex binding region) and an MMTV promotor region. The second LTR region is derived from an MMTV and has a transcription termination site. The second LTR region is contained in the third DNA fragment and is located downstream of the first LTR region across a polylinker into which a foreign gene is to be inserted. With the foreign gene inserted into the polylinker for being expressed within host animal cells, the RNA transcription initiated at the site immediately subsequent to the MMTV promotor region is assuredly brought to a stop when the transcription reaches the transcription termination site on the second LTR region. Protein molecules of a particular type which is specific to the foreign gene used can thus be produced accurately and in stabilized condition within the host animal cells.

The chimeric plasmid vector pMM102 according to the present invention is further characterized in that the first DNA fragment containing the first LTR region also contains a 5'-splicing site located downstream of the first LTR region and that the second DNA fragment is provided downstream of this 5'-splicing site and has a 3'-splicing site located immediately upstream of the polylinker into which a foreign gene is to be inserted, as will also be readily seen from Fig. 3A. By addition of these 5'- and 3'-splicing sites between the first LTR region and the polylinker, the DNA sequence which intervenes between the two splicing sites acts as if it were an intron and is disqualified as a coding sequence. The RNA transcription from the foreign gene as performed within host animal cells may therefore skip over the lengthy DNA sequence spanning from the 5'-splicing site to the 3'splicing site upstream of the polylinker. This means that the proteins which are coded for by the lengthy DNA region thus skipped over are not produced so that the translation of the genetic information from the foreign gene into the form of desired proteins through the RNA transcription will proceed smoothly.

From the foregoing description it will have been appreciated that the present invention provides solution to the various problems which have been inherent in vectors containing a sole MMTV-derived LTR region as hereinbefore pointed out.

The chimeric plasmid vector pMM102 according to the present invention is also characterized in that the vector further includes the fourth DNA fragment having an SV40 promotor region derived from plasmid pSV2gpt and a mycophenolic acid resistant Eco-gpt gene region derived from the plasmid pSV2gpt, as will also be readily seen from Fig. 3A. When a foreign gene is to be expressed within host animal cells, the Eco-gpt gene region of the fourth DNA fragment serves as a gene marker to aid in the selection of the host cell transformed by the chimeric plasmid vector pMM102 according to the present invention. It therefore follows that the transformed host cell acquires a mycophenolic acid resistance stemming from the Eco-gpt gene region and can be isolated with utmost ease. It may also be noted in this instance that the function of the Eco-gpt gene region as the gene marker can not affect the hormonal regulation over the MMTV promotor region since the SV40 promotor region per se is free from the influence of hormones.

The chimeric plasmid vector pMM102 according to the present invention is still further characterized in that the vector further includes the fifth DNA fragment located downstream of the fourth DNA fragment. As will be seen from Fig. 3A, the fifth DNA fragment comprises an ampicillin resistance gene region and a replication origin both of which have been derived from the E. coli plasmid pBR332. Where the chimeric plasmid vector pMM102 having a foreign gene inserted at the polylinker thereof is to be amplified in a cell of E. coli prior to the transformation of host animal cells, the ampicillin resistance gene region of the fifth DNA fragment of the plasmid vector acts as a gene marker in the cell of E. coli so that the cell comes to be blessed with an ampicillin resistance when transformed. This provide ease of isolating the transformed cells.

Still another important aspect of the chimeric plasmid vector pMM102 according to the present invention is that the plasmid vector comprises the first DNA fragment having a first LTR region and a virus packing region located downstream of the first LTR region and capable of packing a virus into globular form and the third DNA fragment located downstream of the first DNA fragment and including a second LTR region as will also be seen from Fig. 3A. A foreign gene is to be inserted into such a plasmid vector at the site of a polylinker intervening between the first and third DNA fragments. The products of replication (which include the foreign gene per se) produced on the DNA sequence ranging from the first LTR region to the second LTR region can therefore be easily recovered in the form of globular viruses. If the globular viruses thus recovered are transfected into a vast number of adequate host animal cells, desired proteins can be produced on a large scale by the expression of foreign genes in the host cells.

In the meantime, the retrovirus-derived chimeric plasmid vector pMM102 according to the present invention stays in stable condition within the chromosome of host animal cells when the plasmid vector is incorporated therein. In addition, the chimeric plasmid vector pMM102 is suitable for the production of a vast amount of gene products by reason of the positive activity of the MMTV promotor region located in one of the LTR regions of the plasmid vector. The MMTV promotor region is sensitive to glucocorticoid hormone and has its activity significantly enhanced by the addition of such a hormone thereto. This characteristic of the MMTV promotor region is useful for regulating the transformative expression of a foreign gene. For example, the transformative expression of a foreign gene within a host animal cell can be regulated with the gene inserted into the chimeric plasmid vector pMM102 for the purpose of assaying the physiologic effects which the particular gene will exhibit within the host animal cell. Where a given foreign gene happens to be potentially detrimental to the host cell, the MMTV promotor region in the chimeric plasmid vector pMM102 will allow such a gene into the host cell in a condition inhibited from being expressed within the cell.

It may be further noted that the polylinker sequence in the chimeric plasmid vector pMM102 according to the present invention has a unique cleavage site for each of the restriction enzymes SalI, SmaI, EcoRI and XbaI. Furthermore, the chimeric plasmid vector pMM102 has two cleavage sites for each of the restriction enzymes BamHI and AccI, one of the two cleavage site being located within the polylinker sequence. This will provide ease of cloning of a DNA fragment digested with any of the restriction enzymes SalI, BamHI, BglII, BclI, SmaI, EcoRI, XbaI, XhoI, AccI, TaqI, Sau3A.

## Synthesis of Chimeric Plasmid Vector pMM102

The features of the chimeric plasmid vector pMM102 provided in accordance with the present invention will be better understood from the following description regarding a typical example of the process of synthesizing the plasmid vector.

### (1) Synthesis of First Intermediate Plasmid pMM1

To 2 μl of plasmid pBR322 (0.5 μg/μl) were added 2 μl of a 10-fold concentrated buffer solution containing 100 mM Tris-HCl (pH 7.6), 70 mM MgCl₂, 70 mM β-mercaptoethanol, and 500 mM NaCl, followed by addition of water to give a total volume of 18 μl. To the aqueous solution thus prepared were added 1 μl of 10 unit/μl BamHI and 1 μl of 8 unit/μl SalI. The resultant mixture was allowed to react at 37°C for 1 hour, was then heated at 65°C for 5 minutes to terminate the reaction and was thereafter subjected to electrophoresis against a 1% agarose gel plate. Thereupon, a band for the 4.1 Kbp DNA was cut from the gel plate and was taken into a dialysis tube containing a Tris-acetate buffer solution (which consisted of 0.04 M Tris-acetate (pH 7.5) and 0.001 M EDTA (ethylenediaminetetraacetate) to extract the DNA therewith through electrophoresis. The DNA preparation accordingly produced in the dialysis tube was then taken out of the tube and was mixed with an equal volume of phenol. The resultant mixture was, upon vigorous shaking, subjected to centrifugation at 15,000 rpm for 5 minutes to collect an aqueous layer containing the DNA. The DNA-containing aqueous layer was mixed with an equal volume of phenol, followed by further centrifugation under the same conditions. Such a procedure was repeated on the resultant DNA-containing aqueous layer after an equal volume of chloroform had been added thereto. The DNA solution obtained as a result of such a repeated procedure was mixed with one-tenth volume of 3 M sodium acetate (pH 7.0) and three volumes of ethanol and was chilled at -70°C for 10 minutes. After cooling, the DNA solution was centrifuged at 15,000 rpm at 0°C for 10 minutes to precipitate DNA and, upon removal of the residual ethanol by evaporation, the resultant DNA preparation was dissolved in 10 μl of water.

On the other hand, 20 μl of 1 μg/μl clone H (Cell, 23, 335-345, 1981) was digested with restriction enzymes BamHI and XhoI (each being of 6 unit/μl activity) and the resultant solution was electrophoresed against a 1% agarose gel plate. A band for the 3.6 Kbp DNA was cut from the gel plate to extract the DNA following the steps used for the separation of DNA using the plasmid pBR322 as the starting material. The DNA thus obtained was dissolved in 10 μl of water, to which solution was added 0.1 μl of BamHI-SalI digested DNA fragment of the plasmid pBR322, 2 μl of a 10-fold concentrated buffer solution containing 660 mM Tris-HCl (pH 7.5), 66 mM MgCl₂, 100 mM DTT (dithiothreitol) and 1 mM ATP, followed by dilution with water to give a total volume of 18 μl. To the aqueous solution thus prepared was added 2 μl of T4 ligase (1.4 unit/μl) and the resultant mixture was allowed to react overnight at 4°C and was thereafter diluted to five folds.

One μl of the DNA preparation obtained in this manner was mixed with 20 μl of competent cell suspension of E. coli HB101 and the mixture was allowed to stand at 0°C for 30 minutes, followed by incubation at 42°C for 2 minutes. After addition of 80 μl of L-broth, the mixture was incubated at 37°C for 30 minutes and was placed onto the surface of an agar plate contain,ng 40 μg/ml of ampicillin. The agar plate was then incubated overnight at 37°C. There were thus obtained colonies, which were assayed for isolation of plasmids pMM1 consisting of the BamHI-SalI digested DNA fragments of plasmid pBR322 linked with the BamHI-XhoI digested DNA fragments of the clone H.

### (2) Synthesis of Second Intermediate Plasmid pMM2

The first intermediate plasmid pMM1 thus produced was cleaved with a restriction enzyme PvuII at the two PvuII cleavage sites of the plasmid. The resultant reaction product was electrophoresed against an agarose gel plate, whereupon a band for the 4.5 Kbp DNA was cut from the gel plate as in step (1). The DNA contained in the cut was recovered therefrom and was ligated with T4 ligase also as in step (1). The resultant circular DNA was cloned by the transformation of E. coli to produce plasmid pMM2 as the second intermediate plasmid. (3) Synthesis of Third Intermediate Plasmid pMM3

The clone H was cleaved with a restriction enzyme BglIII, whereupon a 4.5 Kbp DNA fragment was recovered by agarose gel electrophoresis and was ligated with the BamHI digested DNA fragment of the plasmid pMM2 as in step (1), thereby yielding plasmid pMM3 as the third intermediate plasmid.

(4) Synthesis of Fourth Intermediate Plasmid pMM4

The third intermediate plasmid pMM3 was cleaved with restriction enzymes EcoRI and HindIII and the resultant DNA fragment was precipitated in ethanol and was thereafter dissolved in 21 $\mu$l of water. To the resultant DNA solution were added 2.5 $\mu$l of buffer solution containing 0.5 M Tris-HCl (pH7.2), 0.1 M $MgCl_2$, 1mM DTT and 500 $\mu$g/ml of BSA and 1$\mu$l of deoxyribonucleoside triphosphate mixture (2 mM for each of dATP, dGTP, dCTP and dTTP), followed by addition of 0.5 $\mu$l of 2.6 unit/$\mu$l Klenow enzyme. The resultant mixture was allowed to stand at room temperature for 30 minutes, was then heated at 65°C for 5 minutes, and was treated with 2 $\mu$l of T4 ligase (1.4 unit/$\mu$l) to produce plasmid pMM4 as the fourth intermediate plasmid devoid of EcoRI and HindIII cleavage sites.

(5) Synthesis of Fifth Intermediate Plasmid pMM5

The fourth intermediate plasmid pMM4 was cleaved with a restriction enzyme XbaI and was treated with Klenow enzyme. The resultant fragment was linked to the polylinker sites (multiple restriction enzyme cleavage sites) consisting of a HindIII-EcoRI digested fragment of pi-AN7 which had been treated with Klenow enzyme. The DNA fragment thus obtained was treated also as in step (1) to produce plasmid pMM5 as the fifth intermediate plasmid.

(6) Synthesis of Sixth intermediate Plasmid pMM6

The fifth intermediate plasmid pMM5 was cleaved with restriction enzymes ApaI and KpnI and the resultant fragment was recovered by precipitation in ethanol and was dissolved in 16 $\mu$l of water. To the resultant solution were added 2 $\mu$l of buffer solution containing 0.33 M Tris-acetic acid (pH 7.9), 0.66 M sodium acetate, 0.10 M magnesium acetate, 5.0 mM DTT and 1mg/ml of BSA, followed by addition of 1 $\mu$l of deoxyribonucleoside triphosphate mixture (2 mM for each component)

and 1 $\mu$l of T4 DNA polymerase. The resultant mixture was incubated at 37°C for 5 minutes. The reaction was terminated with addition of 1 $\mu$l of 0.5 M EDTA, whereupon a DNA fragment was extracted by treatment with phenol and was precipitated with ethanol. The resultant DNA fragment was treated as in step (1) to produce plasmid pMM6 as the sixth intermediate plasmid (Fig. 2).

(7) Synthesis of Seventh Intermediate Plasmid pMM7

The sixth intermediate plasmid pMM6 thus obtained was partially digested with a restriction enzyme NdeI and the resultant DNA fragments were extracted by treatment with phenol arid were electrophoresed against an agarose gel plate. A band for the 7.5Kbp DNA was cut from the gel plate and the DNA contained in the cut was extracted by treatment with phenol and was precipitated in ethanol to recover the DNA fragment. The DNA fragment thus recovered was digested with a restriction enzyme HindIII and the resultant DNA fragment was treated with Klenow enzyme and was subjected to treatments similar to those used in step (1) to produce plasmid pMM7 as the seventh intermediate plasmid pMM7.

(8) Synthesis of Eighth Intermediate Plasmid pMM7X1

The seventh intermediate plasmid pMM7 was partially digested with a restriction enzyme NdeI to obtain a 6.5 Kbp DNA fragment as a result of electrophoresis on an agar plate. To this DNA fragment was added 1 $\mu$l of 0.02OD unit/$\mu$l of an Xho linker (commercially available from Takara Shuzo Co., Ltd.) so that the fragment was circularized in a total volume of 20 $\mu$l of the reaction mixture having T4 DNA ligase contained therein. Further using a procedure similar to that taken in step (1), plasmid pMM7X1 was obtained as the eighth intermediate plasmid having the XhoI linker located at the NdeI cleavage site remoter from the polylinker site.

(9) Synthesis of Ninth Intermediate Plasmid pMM8

Ten $\mu$g of plasmid pSV2gt (Science, 209, 1422-1427, 1980) was digested with restriction enzymes PvuII and BamHI to obtain a 2.2 Kbp DNA fragment by agarose gel electrophoresis. The DNA fragment was treated with Klenow enzyme and was linked to an XhoI linker. The resultant DNA fragment was cleaved with a restriction enzyme XhoI whereupon a 2.2 Kbp DNA fragment was recovered by agarose gel electrophoresis and was linked to the XhoI cleavage site of the intermediate plasmid pMM7X1, thus producing plasmid pMM8 as the ninth intermediate plasmid.

## (10) Synthesis of Chimeric Plasmid Vector pMM102

Ten μg of plasmid pCVSVE (Mol. Cell. Biol., 2, 1304-1319, (1982) was digested with restriction enzymes HindIII and PstI whereupon a DNA fragment of about 90 bp was recovered by the electrophoresis conducted against an acrylic amide gel. The intermediate plasmid pMM8 was then partially digested with a restriction enzyme PstI and a DNA fragment of about 8.7 Kbp was recovered by agarose gel electrophoresis. This 8.7 Kbp DNA fragment was further cleaved with a restriction enzyme HindIII and the resultant fragment was linked with the aforesaid DNA fragment of 90 bp, thus producing the chimeric plasmid vector pMM102 according to the present invention.

The various natures of the chimeric plasmid vector pMM102 thus obtained in accordance with the present invention will be further understood from the following Examples.

## Example 1

Tests were conducted to introduce the chimeric plasmid vector pMM102 into an animal cell and to observe the expression of a gene carried by the plasmid within the animal cell.

For this purpose, 100 μg of plasmid pMM102 was dissolved in sterile distilled water. To this solution was added 0.5 ml of 2.5 M $CaCl_2$. The DNA-$CaCl_2$ solution was dropwise added to 2.5 ml of HNP solution in a test tube with bubbling by blowing air into the solution, the HNP solution containing 50 mM HEPES (N-2-hydroxyethyl-piperazine-N'-2-ethansulphonic acid), 280 mM NaCl and 1.5 mM $Na_2HPO_4$ (pH 7.1). The resultant solution was allowed to stand at room temperature for 30 minutes to precipitate the DNA.

On the other hand, mink lung cells (ATCC CCL64) were cultured on a Dulbecco's modified Eagle medium (In vitro, 9, 468-469, 1974) supplemented with 10% fetal calf serum to reach a concentration of $3 \times 10^5$ cells per plate of 60cm diameter (which cell culture corresponds to $10^4$ cells/$cm^2$). To this cell culture was added 0.5 ml per plate of the DNA precipitate produced in the test tube, followed by incubation for 16 hours. To the resultant cell culture was added 0.6 ml of DMSO (glycerol-ethyleneglycol-dimethylsulfoxide) to reach a final concentration of 10%. The culture was then allowed to stand for 30 minutes and thereafter the culture medium was exchanged with new one to continue the incubation. The cells thus yielded were grown on a selective culture medium about fortnight to obtain mycophenolic acid resistant colonies, the medium used consisting of a Dulbecco's modified Eagle medium supplemented with 10% fetal calf serum, 15 μg/ml of hypoxanthine, 10 μg/ml of thymidine, 5 μg/ml of glycine, 2 μg/ml of aminopterin, 25 μg/ml of mycophenolic acid, and 250 μg/ml of xanthine. The growth of such resistive colonies shows that the plasmid pMM102 was incorporated into the mink lung cells and an Eco-gpt gene was expressed in each of the cells.

## Example 2

Tests were then conducted to examine the glucocorticoid hormone denendency of the agent of the promotor region of the MMTV.

For this purpose, fifteen strains of the mycophenolic acid resistant cells obtained by Example 1 were cultured individually of one another. The resultant cell cultures were grouped into two different categories whereby the cell cultures of one category were incubated with addition of $10^{-6}$ M dexamethasone thereto and the cell cultures of the other category were incubated without addition of dexamethasone. In 28 hours, a total quantity of RNA was extracted from the resultant cell cultures using the guanidium-CsCl method. A DNA fragment was obtained by cleaving the plasmid pMM102 downstream of the MMTV promotor region with a restriction enzyme BglII and was labeled with isotope $^{32}$P. Analysis was made using a hybridization method into the quantity of the RNA complementary to the DNA fragment resulting from the BglII-digested plasmid pMM102, revealing that the transcription rate had increased about ten times for two of the strains to which dexamethasone had been added. This means that the transcription conducted by the MMTV promotor region is dependent on the hormone used.

## Example 3

Tests were further conducted to observe the expression of a CAT gene (choramphenicol resistance gene) within an animal cell.

Plasmid pBR325 (Gene, 14, 289, 1981) was cleaved with a restriction enzyme TaqI to obtain a DNA fragment containing CAT gene. The DNA fragment was treated with Klenow enzyme and a SalI linker was linked to the resultant fragment, which was then cloned at the SalI cleavage site on a polylinker of the plasmid vector pMM102. A DNA fragment was then selected which had a CAT gene inserted into the polylinker in a positive direction with respect to the MMTV promotor region and was introduced into a mink lung cell as in a manner used in Example 1. After incubation for 48 hours, the cell was suspended in 0.25 M Tris buffer solution and the suspension thus prepared was subjected to ultra-sonication for 1 minute to cause the cell to disrupt, followed by centrifugation at 15,000 rpm for 5 minutes to collect the supernatant. To this supernatant were added $^{14}$C-labeled chloramphenicol and acetyl-CoA. After incubation at 30°C for 60 minutes and subsequent extraction of the liquid substance of the cell into ethyl acetate, an organic layer of ethyl acetate was dried and the resultant substance was dissolved in 10 $\mu$l of ethyl acetate. The preparation thus obtained was subjected to silica-gel thin-layer chromatography to develop using a 95:5 (v/v) chloroform-methanol mixture. After the development and drying procedures, analysis was made into the chromatogram generated by an x-ray film held in contact with the resultant preparation by means of a radioautography method. The analysis revealed that chloramphenicol was acetylated in the extracted liquid substance of the cell into which the CAT gene had been introduced. This means that the CAT gene was expressed within the cell.

**Claims**

1. A chimeric plasmid vector which comprises
    (a) a first DNA (deoxyribonucleic acid) fragment having a first LTR (long terminal repeat) region derived from a mouse mammary tumor virus (MMTV) and a 5'-splicing site located downstream of the first LTR region, the first LTR region comprising
        (a-1) a hormone dependent region, and
        (a-2) a MMTV promotor region located downstream of the hormone dependent region;
    (b) a second DNA fragment located downstream of said first DNA fragment and having a 3'-splicing site derived from plasmid pCVSVE;
    (c) a polylinker derived from plasmid pi-AN7 and located downstream of said second DNA fragment;
    (d) a third DNA fragment located downstream of said polylinker and having a second LTR region derived from said MMTV, the second LTR comprising
        (d-1) a transcription termination site;
    (e) a fourth DNA fragment located downstream of said third DNA fragment, the fourth DNA fragment comprising
        (e-1) an SV40 promotor region derived from plasmid pSV2gpt, and
    (f) a fifth DNA fragment located downstream of said fourth DNA fragment and between said first DNA fragment and said fourth DNA fragment, the fifth DNA fragment comprising
        (f-1) an ampicillin resistance gene region derived from plasmid pBR322, and
        (f-2) a replication origin derived from said plasmid pBR322.

2. A chimeric plasmid vector according to claim 1 in which said first DNA fragment consists of a DNA fragment of clone H cleaved with restriction enzymes BamHI and XhoI, said clone H being derived by cloning an MMTV at a EcoRI cleavage site of a lambda-phage vector lambda-gtWES lambda-B.

3. A chimeric plasmid vector according to claim 1 or 2 in which said third DNA fragment consists of a DNA fragment of clone H cleaved with restriction enzyme Bg1II.

4. A chimeric plasmid vector according to any one of claims 1-3 in which said second DNA fragment having said 3'-splicing site contained therein consists of a DNA fragment of plasmid pCVSVE cleaved with restriction enzymes PstI and HindIII.

5. A chimeric plasmid vector according to any of the preceding claims in which said fourth DNA fragment having said Eco-gpt gene region contained therein consists of a DNA fragment of plasmid SV2gpt cleaved with restriction enzymes PvuII and BamHI.

6. A chimeric plasmid vector, denoted pMM102 herein, and deposited in E. Coli K-12 under FERM BP-1157, the restriction enzyme map of which is as shown in Figures 3 and 3A of the accompanying drawings.

7. A chimeric plasmid vector claimed in any one of the preceding claims which has a foreign gene inserted in its polylinker (c).

8. A method employing a vector to convey a foreign gene into an animal host cell, to retain and express the foreign gene therein, in which method there is used as vector a chimeric plasmid vector claimed in claim 7.

9. An animal host cell transformed by containing a chimeric plasmid vector claimed in claim 7.

10. A method of producing a protein by animal cells in which the animal cells are animal cells transformed by containing a chimeric plasmid claimed in claim 7 wherein the foreign gene codes for the protein.

**Revendications**

1. Vecteur plasmidique chimérique qui comprend
    (a) un premier fragment d'ADN (acide désoxyribonucléique) ayant une première région LTR (longue répétition terminale) provenant d'un virus de tumeur mammaire de souris (MMTV) et un site d'épissage 5' situé en aval de la première région LTR, la première région LTR comprenant
        (a-1) une région hormono-dépendante, et
        (a-2) une région de promoteur de MMTV située en aval de la région hormono-dépendante ;
    (b) un deuxième fragment d'ADN situé en aval dudit premier fragment d'ADN et ayant un site d'épissage 3' provenant du plasmide pCVSVE ;
    (c) un polyadaptateur provenant du plasmide pi-AN7 et situé en aval dudit deuxième fragment d'ADN ;
    (d) un troisième fragment d'ADN situé en aval dudit polyadaptateur et ayant une seconde région LTR provenant dudit MMTV, la seconde région LTR comprenant
        (d-1) un site de terminaison de transcription ;
    (e) un quatrième fragment d'ADN situé en aval dudit troisième fragment d'ADN, le quatrième fragment d'ADN comprenant :
        (e-1) une région de promoteur de SV40 provenant du plasmide pSV2gpt, et
    (f) un cinquième fragment d'ADN situé en aval dudit quatrième fragment d'ADN et entre ledit premier fragment d'ADN et ledit quatrième fragment d'ADN, le cinquième fragment d'ADN comprenant :
        (f-1) une région de gène de résistance à l'ampicilline provenant du plasmide pBR322, et
        (f-2) une origine de réplication provenant dudit plasmide pBR322.

2. Vecteur plasmidique chimérique selon la revendication 1, dans lequel ledit premier fragment d'ADN consiste en un fragment d'ADN de clone H clivé par les enzymes de restriction *Bam*HI et *Xho*I, ledit clone H étant dérivé par clonage d'un MMTV au site de clivage *Eco*RI d un vecteur dérivé de phage lambda, lambda-gtWES.lambda-B.

3. Vecteur plasmidique chimérique selon la revendication 1 ou 2, dans lequel ledit troisième fragment d'ADN consiste en un fragment d'ADN de clone H clivé par l'enzyme de restriction *Bgl*II.

4. Vecteur plasmidique chimérique selon l'une quelconque des revendications 1 à 3, dans lequel ledit deuxième fragment d'ADN contenant ledit site d'épissage 3' consiste en un fragment d'ADN de plasmide pCVSVE clivé par les enzymes de restriction *Pst*I et *Hin*dIII.

5. Vecteur plasmidique chimérique selon l'une quelconque des revendications précédentes, dans lequel ledit quatrième fragment d'ADN contenant ladite région de gène *Eco*-gpt consiste en un fragment d'ADN du plasmide pSV2gpt clivé par les enzymes de restriction *Pvu*II et *Bam*HI.

6. Vecteur plasmidique chimérique, dénommé ici pMM102 et déposé dans *E. coli* K-12 sous le numéro FERM BP-1157, dont la carte de restriction enzymatique est telle que représentée sur les Figures 3 et 3A des dessins annexés.

7. Vecteur plasmidique chimérique selon l une quelconque des revendications précédentes, qui comporte un gène étranger inséré dans son polyadaptateur (c).

8. Procédé faisant usage d'un vecteur pour transporter un gène étranger dans une cellule-hôte animale, de façon à retenir et exprimer le gène étranger dans celle-ci, procédé dans lequel on utilise comme vecteur un vecteur plasmidique chimérique revendiqué dans la revendication 7.

9. Cellule-hôte animale transformée du fait qu'elle contient un vecteur plasmidique chimérique revendiqué dans la revendication 7.

10. Procédé de production d'une protéine par des cellules animales, dans lequel les cellules animales sont des cellules animales transformées du fait qu'elles contiennent un plasmide chimérique revendiqué dans la revendication 7 dans lequel le gène étranger code pour la protéine.

**Patentansprüche**

1. Chimärer Plasmidvektor, **dadurch gekennzeichnet**, daß er
    (a) ein erstes DNA (deoxyribonucleic acid)-Fragment, das einen ersten LTR (long terminal repeat)-Bereich, der von einem Maus-Mamma-Tumor-Virus (MMTV) abstammt, und einen 5'-Spleißort, der stromabwärts zum ersten LTR-Bereich liegt, enthält, der erste LTR-Bereich enthaltend
        (a-1) einen hormonabhängigen Bereich, und
        (a-2) einen MMTV-Promotorbereich,

der stromabwärts zum hormonabhängigen Bereich liegt;

(b) ein zweites DNA-Fragment, das stromabwärts zum ersten DNA-Fragment liegt und das einen 3'-Spleißort enthält, der aus dem Plasmid pCVSVE stammt;

(c) einen Polylinker, der aus dem Plasmid pi-AN7 stammt, und der stromabwärts zum zweiten DNA-Fragment liegt,

(d) ein drittes DNA-Fragment, das stromabwärts zum Polylinker liegt, und das einen zweiten LTR-Bereich aufweist, der von dem MMTV stammt, der zweite LTR-Bereich enthaltend

    (d-1)     einen Transkriptionsterminatorort;

(e) ein viertes DNA-Fragment, das stromabwärts zum dritten DNA-Fragment liegt, das vierte DNA-Fragment enthaltend

    (e-1)     einen SV40-Promotorbereich, der aus dem Plasmid pSVgpt stammt, und

(f) ein fünftes DNA-Fragment, das stromabwärts zum vierten DNA-Fragment und zwischen dem ersten DNA-Fragment und diesem vierten DNA-Fragment liegt, das fünfte DNA-Fragment enthaltend

    (f-1)     einen Ampicillin-Resistenz-Genbereich, der von dem Plasmid pBR322 stammt, und

    (f-2)     einen Replikationsursprung, der von diesem Plasmid pBR322 stammt

enthält.

2.   Chimärer Plasmidvektor gemäß Anspruch 1, **dadurch gekennzeichnet**, daß das erste DNA-Fragment aus einem DNA-Fragment des Klons H besteht, das mit dem Restriktionsenzym BamHI und XhoI gespalten wurde, wobei der Klon H durch Klonierung eines MMTV in eine EcoRI-Schnittstelle eines Lambda-Phagen Vektor Lambda-gtWES Lambda-B erhalten wurde.

3.   Chimärer Plasmidvektor gemäß Anspruch 1 oder 2, **dadurch gekennzeichnet**, daß das dritte DNA-Fragment aus einem DNA-Fragment des Klons H besteht, das mit dem Restriktionsenzym Bg1II gespalten wurde.

4.   Chimärer Plasmidvektor gemäß einem der vorhergehenden Ansprüche 1 bis 3, **dadurch gekennzeichnet**, daß das zweite DNA-Fragment mit dem darin enthaltenen 3'-Spleißort aus einem DNA-Fragment des Plasmids pCVSVE besteht, das mit den Restriktionsenzymen PstI und HindIII gespalten wurde.

5.   Chimärer Plasmidvektor nach einem der vorhergehenden Ansprüche, **dadurch gekennzeichnet**, daß das vierte DNA-Fragment mit dem darin enthaltenen Eco-gpt-Genbereich aus einem DNA-Fragment des Plasmids SV2gpt besteht, das mit den Restriktionsenzymen PvuII und BamHI gespalten wurde.

6.   Chimärer Plasmidvektor, bezeichnet als pMM102 und hinterlegt in E. coli K-12 unter der Nummer FERM BP-1157, dessen Restriktionsenzymkarte in Abbildung 3 und Abbildung 3A der begleitenden Zeichnungen gezeigt wird.

7.   Chimärer Plasmidvektor nach einem der vorhergehenden Ansprüche, **dadurch gekennzeichnet**, daß er ein fremdes Gen in seinem Polylinker (C) enthält.

8.   Ein Verfahren unter Verwendung eines Vektors, um fremde Gene in tierische Gastzellen einzubringen, um die fremden Gene darin zu bewahren und zu exprimieren, **dadurch gekennzeichnet**, daß als Vektoren chimäre Plasmidvektoren gemäß Anspruch 7 verwendet werden.

9.   Eine tierische Gastzelle, **dadurch gekennzeichnet**, daß sie einen chimären Plasmidvektor gemäß Anspruch 7 enthält und dadurch transformiert wurde.

10. Verfahren zur Herstellung von Protein durch tierische Zellen, **dadurch gekennzeichnet**, daß die tierischen Zellen einen chimären Plasmidvektor gemäß Anspruch 7 enthalten und dadurch transformiert sind, wobei die fremden Gene für das Protein kodieren.

12

FIG. 2

# FIG. 3

# FIG. 3A

## F i g 4

AAGCTT|CTAG|ATGATCT|CTGCAG|GTCGAC|GGATCC|CCGG|GAATTC

TTCGAA|GATC|TCTAGTA|GACGTC|CAGCTG|CCTAGG|GGCC|CTTAAG

Hind III | Xba I | Bgl II | Pst I | Sal I | BamH I | Sma I | EcoR I

## F i g 5

C  C  T  C  G  A  G  G

G  G  A  G  C  T  C  C